# EUROPEAN PATENT APPLICATION

(11) **EP 3 636 255 A1**
(43) Date of publication of application: **15.04.2020**
(21) Application number: 18199192.8
(22) Date of filing: 08.10.2018
(51) Int. Cl.: A61K 31/133, A61L 2/16, A61K 31/164, A61P 31/04, A61P 31/12

(54) **USE OF DEOXYSPHINGOLIPIDS AS ANTI-TOXINS AND ANTI-VIRALS**

(71) Applicant: UNIVERSITE DE GENEVE, 1211 Geneva 4 (CH); Ecole Polytechnique Fédérale de Lausanne (EPFL), 1015 Lausanne (CH)
(72) Inventor: RIEZMAN, Howard S., 1285 Athenaz (Avusy) (CH); HANNICH, Joerg Thomas, 74240 Gaillard (FR); GALIH, Augustinus, 1202 Genève (CH); GRUENBERG, Jean, 1270 Trelex (CH); VAN DER GOOT, Françoise Gisou, 1270 Trelex (CH)
(74) Representative: reuteler & cie SA

(57) **Abstract**

The present invention relates 1-deoxysphingolipids, as well as pharmaceutically acceptable salts thereof, for use in the prevention and/or treatment of intoxication or viral infections, in particular 1-deoxyceramide or a 1-deoxysphingoid base prodrug thereof. This invention provides pharmaceutical compositions comprising 1-deoxysphingolipids of the invention and uses thereof.

## Description

### Field of the Invention

The present invention relates to agents belonging to the sphingolipid family useful for interfering with intoxication, toxin-mediated infection and viral infection and their use as anti-toxins and anti-virals.

### Background of the Invention

Plant or bacterial toxins, as well as infectious viruses, cause severe health problems. Most of the strategies to counter toxins as well as viruses depend upon protein-based strategies, for instance vaccination or neutralizing antibodies.

Since a large group of bacterial and plant toxins bind to cells by one moiety whereas another moiety with enzymatic activity enters the cytosol and exerts their action, those have to be endocytosed before translocation to the cytosol. Several bacterial toxins, such as diphtheria toxin and anthrax toxin, enter the cytosol in response to the low pH found in endosomes, whereas other toxins, such as the plant toxin ricin, and the bacterial cholera and Shiga toxins are transported not only to endosomes, but also retrogradely through the Golgi apparatus and to the endoplasmic reticulum before the enzymatically active part enters the cytosol (Sandvig and van Deurs, 2002, FEBS Lett., 529(1), 49-53). Also many virus infections use the endocytic pathway where after attachment to the cells, virions undergo clathrin-mediated endocytosis followed by proteolytic disassembly of the capsid and penetration to the cytoplasm for introducing their genetic material to the cell interior (Schulz et al., 2012, J. Virol., 86(23): 12665-12675*;* Cossart et al., 2014, Cold Spring Harb Perspect Biol., 6(8): a016972*).*

Among the toxins and viruses that cause major public health concern, a large number of those use a complex mechanism of intoxication or infection of the host through the endocytic pathway, where the chaperonin containing TCP1/T-complex protein-1 (TCP1)-ring complex (CCT/TRiC) plays an important role. CCT/TRiC is a ubiquitous, hetero-oligomeric complex formed by two eight-membered rings composed of paralogous units (CCT1-CCT8). The role of CCT/TRiC in the replication of eukaryotic viruses such as hepatitis B and C, Epstein Barr virus, rabies virus, influenza virus and reoviruses has been described (Knowlton et al., 2018, Nat Microbiol., 3(4): 481-493). In addition, CCT/TRiC was shown as being required for efficient delivery of active toxins to the cytosol of some toxins such as that of *Bacillus anthracis* (anthrax toxin) (Slater et al., 2013, Proc Natl Acad Sci U S A., 110(24): 9932-9937) and Pseudomonas exotoxin (Moreau et al, 2011, Dev. Cell 21: 231-244) and most recently also *Clostridium difficile* toxins A and B *(*Steinemann et al., 2018, Proc Natl Acad Sci U S A., pii: 201807658*).* CCT/TRiC is mainly known to play a role in the folding of newly synthesized actin and tubulin monomers but is also essential to fold many other globular proteins in the cytosol.

Sphingolipids are made in all eukaryotic cells and can also be synthesized by certain bacteria (Merrill et al., 2009, J Lipid Res., 50 Suppl:S97-102). The sphingolipid biosynthesis pathway begins with the synthesis of 3-ketodihydrosphinganine by the enzyme serine palmitoyltransferase (SPT). The normal substrates of this enzyme are serine and palmitoyl-CoA. 3-ketodihydrosphinganine is then reduced to sphinganine which can be acylated to form dihydroceramide (Merrill, 2011, Chem Rev., 111(10):6387-422) (Hannun and Obeid, 2018, Nat. Rev. Mol. Cell Biol. 19(3): 175-191). The dihydroceramide can then be desaturated and/or converted to more complex sphingolipids by headgroup addition to the 1-hydroxyl group of the sphingoid base moiety. In an inherited disease (hereditary sensory and autonomic neuropathy, type IA) or under conditions where serine is depleted, SPT can also accept alanine in the place of serine producing 1-deoxy, 3-ketosphinganine, which is then converted to 1-deoxysphinganine, 1-deoxydihydroceramide and 1-deoxyceramide (Penno et al., 2010, J Biol Chem., 285(15):11178-87*;* Gable et al., 2010, J Biol Chem., 285(30):22846-52*;* Esaki et al. 2015, J Biol Chem., 290(23):14595-609). Ceramides formed from 1-deoxysphinganine are present in normal cells at low levels, but represent a minority over the larger amounts of conventional ceramides. Subsequent steps in sphingolipid biosynthesis are blocked due to the absence of the 1-hydroxyl group in the sphingoid base moeity. Sphingolipids are essential for growth of all eukaryotic cells but the functions of 1-deoxysphingolipids are not known.

Currently, there are few small molecule inhibitors of toxins and viral infections and therefore there exists an evident medical need for efficient anti-toxin and anti-viral agents since economic and public health implications of toxin and virus induced diseases are growing with the increase of population density and related contamination risks in addition to the development of biological weapons.

### Summary of the Invention

The present invention is directed to the finding of a family of 1-deoxysphingolipids which act as anti-toxins and anti-virals by interfering with toxin action and virus infection via the endocytic pathway, well below toxic concentrations. In particular, the invention is based on the findings that treatment of tissue culture cells with a 1-deoxysphingolipid, in particular 1-deoxysphinganine, strongly inhibits virus replication and anthrax toxin toxicity at concentrations where cellular toxicity is not observed. It is believed that this type of agent acts via the formation of 1-deoxydihydroceramide which interferes with the toxin entry and virus infection processes via the endocytic pathway by inactivation of the CCT/TRiC. It has been shown that different types of bacterial toxins and viral infections require the activity of CCT/TRiC which folds many cytosolic proteins.

It is an object of the invention to provide new therapeutic agents useful for increasing the efficacy of inhibitors of toxin action and viral infection or useful alone as anti-toxin and anti-viral agents for preventing and/or treating toxin-mediated infection or intoxication involving bacterial or plant toxins or infection by viruses acting through the endocytic pathways.

A first aspect of the invention provides a 1-deoxysphingolipid, in particular a 1-deoxyceramide or a 1-deoxysphingoid base prodrug thereof, as well as pharmaceutically acceptable salts thereof, for use in the prevention and/or treatment of toxin-mediated infection or intoxication involving bacterial or plant toxins or viral infections.

Another aspect of the invention resides in a use of a compound according to the invention for the preparation of a pharmaceutical composition, in particular for the prevention and/or treatment of toxin-mediated infection or intoxication involving bacterial or plant toxins or viral infections.

Another aspect of the invention provides a pharmaceutical composition comprising at least one compound according to the invention and a pharmaceutically acceptable carrier, diluent or excipient thereof.

Another aspect of the invention relates to a pharmaceutical composition comprising at least one compound according to the invention in combination with at least one agent useful in the preventing and/or treating of toxin-mediated infection or intoxication involving bacterial or plant toxins or infection by viruses.

Another aspect of the invention is a method for preventing and/or treating a subject suffering from or at risk of suffering from toxin-mediated infection or intoxication involving bacterial or plant toxins or infection by viruses, comprising administering a compound according to the invention or a pharmaceutical formulation thereof in a subject in need thereof.

Another aspect of the invention relates to a use of a 1-deoxysphingolipid or a composition thereof for sterilizing and/or decontaminating or preventing from contamination with toxins or viruses, medical material intended for medical, aesthetic or hygiene practice or preparations intended for human or veterinary use such as for tissue or cell preparations.

A method of sterilisation and/or decontamination or preventing contamination with toxins or viruses, medical material intended for medical, aesthetic or hygiene practice or preparations intended for human or veterinary use comprising the step of contacting said material, room or preparations with a 1-deoxysphingolipid, or a composition thereof.

An *ex vivo* method of inhibiting virulence of bacteria and/or viruses, said method comprising the step of contacting a material or a solution at risk being contaminated by said bacteria and/or viruses with a 1-deoxysphingolipid, or a composition thereof.

Further objects and advantageous aspects of the invention will be apparent from the claims and/or from the following detailed description of embodiments of the invention with reference to the annexed drawings.

### Brief description of the drawings

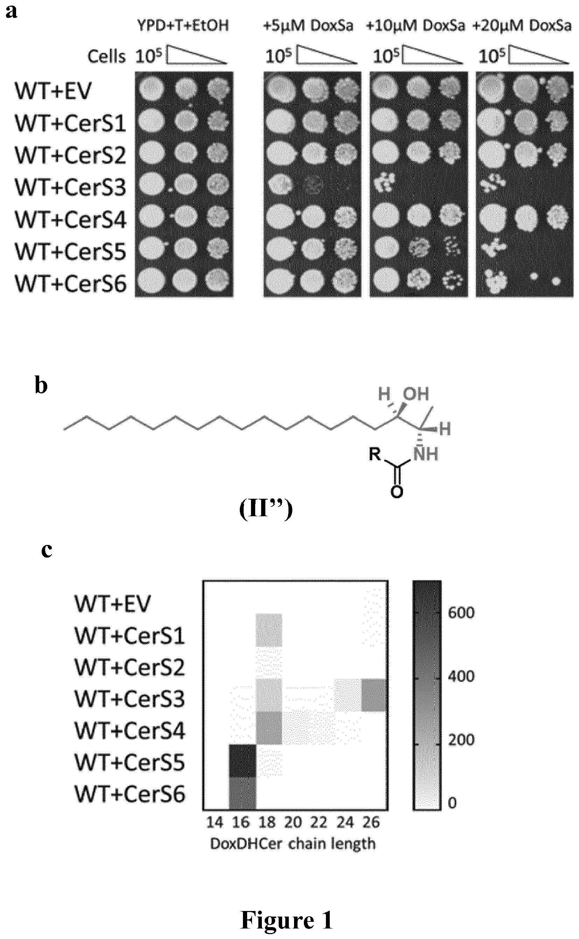

**Figure 1** shows the effects on yeast cells of a 1-deoxysphingoid base prodrug of the invention, 1-deoxysphinganine **(DoxSa) (1)** through the conversion to its active corresponding ceramide as described in Example 1. **a:** represents the sensitivity of WT yeast cells with an empty vector (WT+EV) or one expressing each of the six mammalian ceramide synthases (WT+CerS1-S6) to various concentrations of the 1-deoxysphingolipid compared to control (no 1-deoxysphingolipid, left). **b:** represents a typical structure of Formula (II") of a yeast 1-deoxydihydroceramide (DoxDHCer) from *S. cerevisiae* containing non-canonical DoxSa (in grey) where **R** is an alkyl chain with 13 to 25 carbons which can be hydroxylated;
**c:** represents DoxDHCer species produced by WT cells expressing mammalian ceramide synthases following a 1.5 h treatment with 2.5 µM of DoxSa.

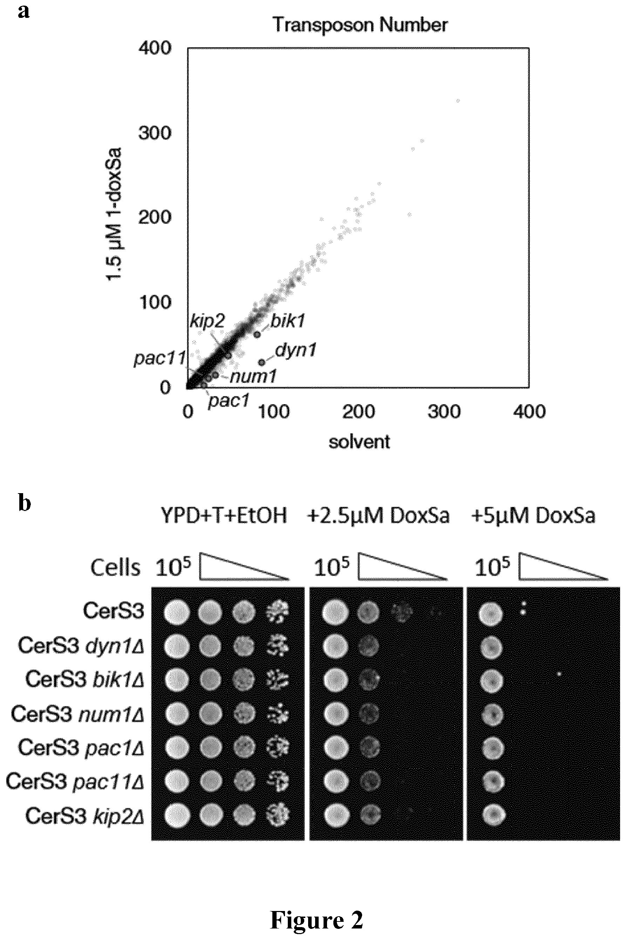

**Figure 2** represents results from Saturated Transposon Analysis in Yeast (SATAY) in response to 1.5 µM of 1-deoxysphinganine **(DoxSa) (1)** using a library of yeast cells expressing mammalian ceramide synthase 3 (CerS3) as described in Example 2. **a:** null mutants in the library that are hypersensitive to 1-deoxydihydroceramide (DoxDHCer) evaluated by comparing the number of transposon insertion sites per gene before and after the DoxSa treatment; **b:** represents the sensitivity to DoxDHCer of CerS3 cells lacking the indicated genes.

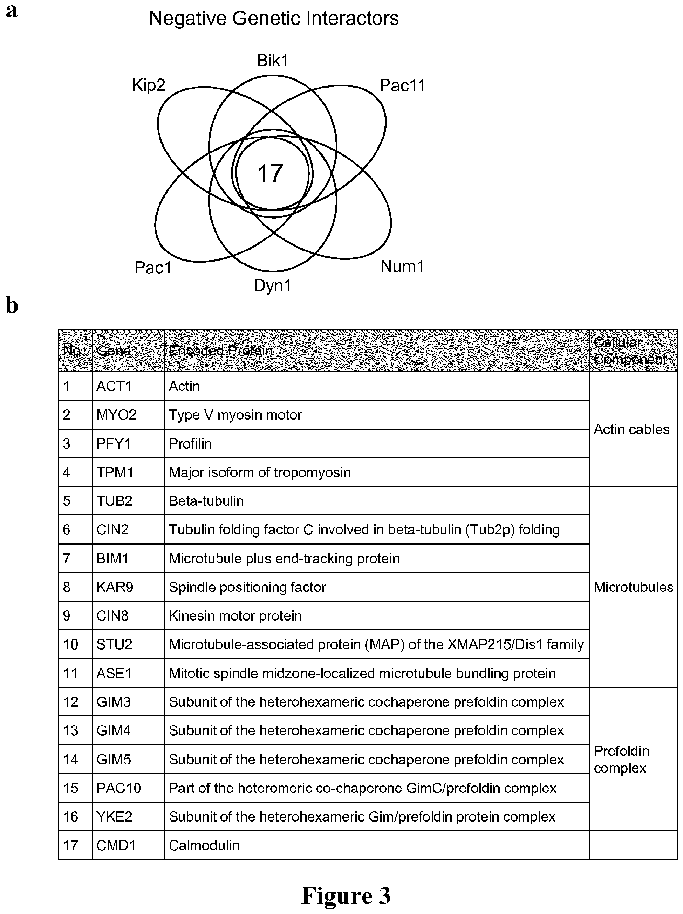

**Figure 3** represents the results of negative genetic interaction analysis of hypersensitive mutants revealed by SATAY as described in Example 2. **a:** Venn diagram of synthetic genetic interactions for factors that have been identified as hypersensititive to 1-deoxysphinganine/1-deoxydihydroceramide where 17 common interactions have been identified. **b:** List of the 17 common negative genetic interactors which identifies the involvement of the folding pathway of cytosolic globular proteins since both the folding machinery (prefolding complex) as well as two different cytoskeletal structures (actin cables and microtubules) appear in this list.

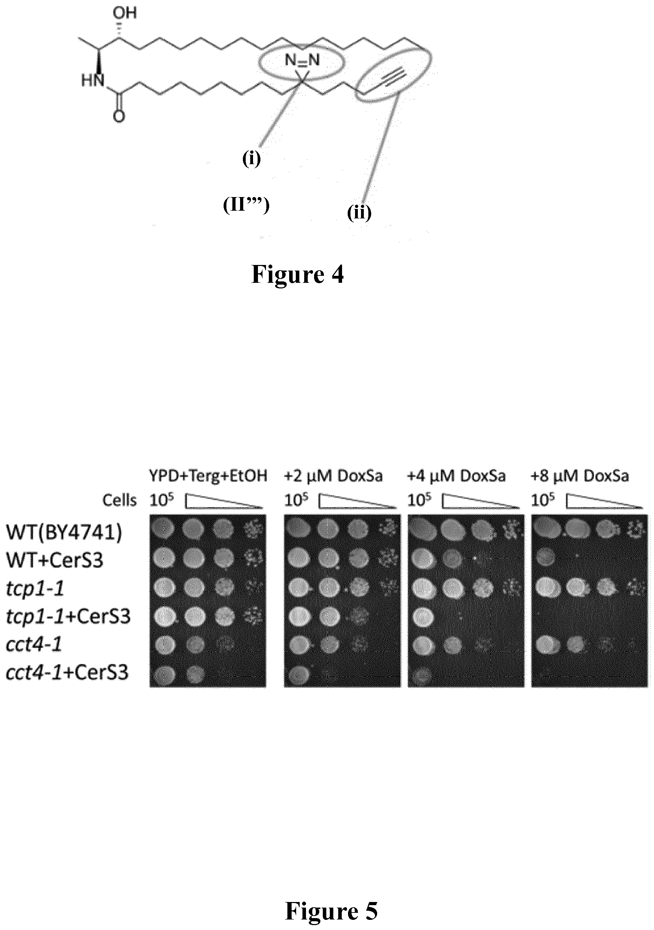

**Figure 4** represents the structure of Formula (**II"'**), an example of a synthetic 1-deoxydihydroceramide according to Formula (II) which is used as a photo-cross-linkable 1-deoxydihydroceramide bio-orthogonal probe for chemoproteomic analysis described in Example 2, comprising **(i)** an alkyne group to allow functionalization by biotinylation by click chemistry and **(ii)** a diazirine group to allow photo-activated cross-linking to protein targets.
**Figure 5** represents the sensitivity of CCT/TRiC temperature-sensitive mutants to 1-deoxydihydroceramide compared to wildtype (WT), as described in Example 2.

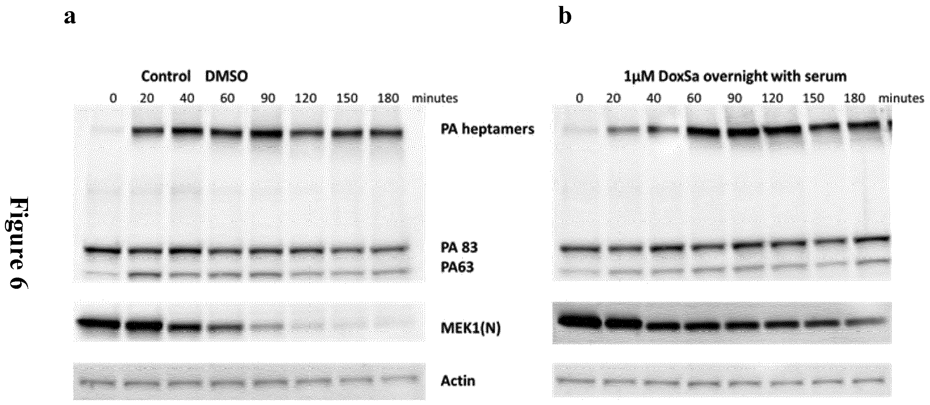

**Figure 6** represents a western blot analysis of RPE-1 cells incubated with solvent control (DMSO) or 1 µM 1-deoxysphinganine **(DoxSa)** overnight and exposed to anthrax toxin mix of protective antigen (PA) and lethal factor (LF) as described in Example 3. **a:** control; **b:** DoxSa treatment. In both control and DoxSa treatment conditions, PA is processed from PA83 to PA63 and heptameric PA pores form. In contrast, while in control conditions LF cleaves MAP kinase kinase (MEK1) within 90 to 120 minutes, DoxSa treatment prevents LF activity and therefore MEK1 is stabilized as monitored by western blot using an antibody against the N-terminus of MEK1 (MEK1(N)). Immuno-detection of actin was used for normalization.

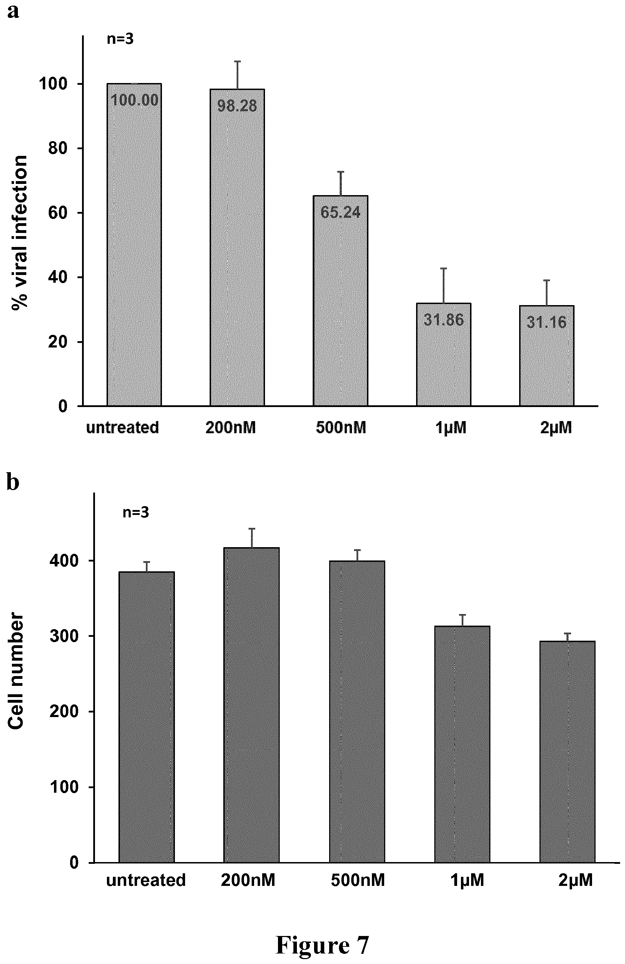

**Figure 7** represents the effects of C18 1-deoxysphinganine (DoxSa, **1**) on VSV infections as shown by **(a)** the percentage of infection of HeLa cells treated with different concentrations (0.2-2 µM) of DoxSa overnight and incubated with infectious VSV viral particles encoding for a GFP fusion of the P-protein as measured by fluorescence and by (**b**) the cell number quantified by DAPI nuclei count as described in Example 3. After 3 hours of incubation infection was quantified as GFP-positive cells, values are normalized to 100% for untreated infections. Concentration dependent inhibition of viral infection can be observed. **b:** Quantification of cell number by DAPI-based nuclei count. DoxSa starts to show some toxicity at 1µM. Values are averages from n=3 independent experiments with each 2 or 3 biological replicates, error bars are standard deviations.

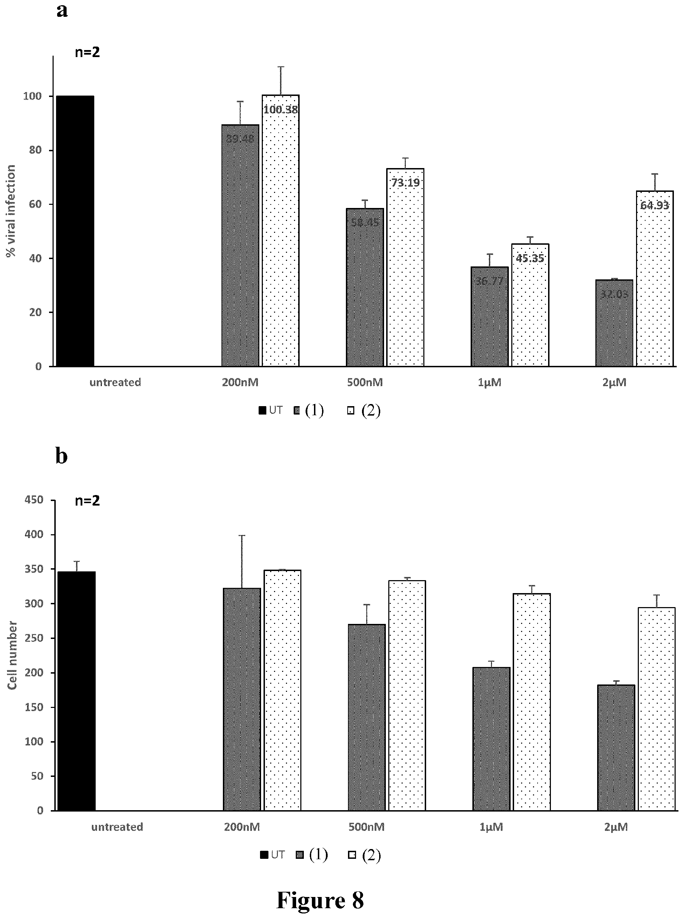

**Figure 8** represents the effects of C18 1-deoxysphinganine **(1)** vs. ω-1 alkyne-1-deoxysphinganine (**2**) on VSV infections as shown by **(a)** the percentage of infection of HeLa cells treated with different concentrations (0.2-2 µM) of **(1)** and **(2)** overnight and incubated with infectious VSV viral particles encoding for a GFP fusion of the P-protein as measured by fluorescence and by (**b**) the cell number quantified by DAPI nuclei count as described in Example 3. After 3 hours of incubation, infection was quantified as GFP-positive cells, values are normalized to 100% for untreated infections. Values are averages from n=2 biological replicates, error bars are standard deviations.

### Detailed Description of the invention

The term "intoxication or viral infections" as defined herewith refers to all stages of contamination with a toxin from bacteria or plant or of an infection by a virus, wherein said toxin or virus uses an endocytic pathway for invading host cells and/or for propagation. Examples of intoxication according to the invention are intoxication by bacterial toxins such as from *Bacillus anthracis* (Anthax), *Vibrio cholerae* (Cholera), by *S. dysenteriae, Clostridium difficile,* and the shigatoxigenic serotypes of *Escherichia coli* (STEC) (Shiga toxin) and by plant toxins such as from *Ricinus communis* (castor beans) (ricin). Examples of viral infections according to the invention are viral infections from influenza virus, reoviruses such as Rotavirus, Hepatitis B, Hepatitis C, Epstein Barr virus, Rabies virus, Mason Pfizer monkey virus (simian retrovirus), Vesicular Stomatitis Virus and relatives (Rhabdoviridae), Flaviviridae like Dengue virus, foot and mouth, cattle, fish and other non-enveloped viruses. The term "subject" as used herein, refers to a human or a non-human mammal, such as non-human primate (e.g. chimpanzees and other apes and monkey species), a farm animal (e.g. cattle, sheep, pigs, goats and horses), a domestic mammal (e.g. dogs and cats), or a laboratory animal (e.g. rodents, such as mice, rats and guinea pigs).

The term "efficacy" of a treatment according to the invention can be measured based on changes in the course of disease in response to a use or a method according to the invention. For example, the efficacy of a treatment according to the invention can be measured by its impact on signs or symptoms of illness. A response is achieved when the subject experiences partial or total alleviation, or reduction of unwanted symptoms of illness. According to a particular embodiment, the efficacy can be measured through the assessment of toxin target cleavage or viral replication after infection. For example, the efficacy of a toxin or anti-viral treatment according to the invention can be monitored by following the effect on map kinase kinase 1 cleavage or by the improvement of cell survival, tissue damage and patient survival. As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease.

The term "alkoxycarbonyl" refers to the group -C(O)OR where R includes "C₁-C₆ alkyl", "aryl", "heteroaryl" , "aryl C₁-C₆ alkyl", "heteroaryl C₁-C₆ alkyl" or "heteroalkyl".

The term "alkoxycarbonyl C₁-C₆ alkyl" refers to C₁-C₆ alkyl groups having an alkoxycarbonyl substituent, including 2-(benzyloxycarbonyl)ethyl and the like.

The term "amino" refers to the group -NRR' where R and R' are independently H , "C₁-C₆ alkyl", "aryl", "heteroaryl", "C₁-C₆ alkyl aryl", "C₁-C₆ alkyl heteroaryl," "cycloalkyl," or "heterocycloalkyl," and where R and R', together with the nitrogen atom to which they are attached, can optionally form a 3-8-membered heterocycloalkyl ring.

Unless otherwise constrained by the definition of the individual substituent, the term "substituted" refers to groups substituted with from 1 to 5 substituents selected from the group consisting of hydroxy, amino, amide, carbonyl (-C(O)-) and alkoxycarbonyl.

The term "pharmaceutical formulation" refers to preparations which are in such a form as to permit biological activity of the active ingredient(s) to be unequivocally effective and which contain no additional component which would be toxic to subjects to which the said formulation would be administered.

### Compounds of the invention

According to one aspect, is provided a 1-deoxysphingolipid, in particular a 1-deoxyceramide or a 1-deoxysphingoid base prodrug thereof, as well as pharmaceutically acceptable salts thereof, for use in the prevention and/or treatment of toxin-mediated infection or intoxication involving bacterial or plant toxins or viral infections.

According to a particular aspect, a 1-deoxysphingoid base prodrug of a 1-deoxyceramide is a 1-deoxysphingoid base which can be transformed into a (dihydro-) ceramide by ceramide synthases (CerS) such as mammalian ceramide synthases 3, 5, and 6.

According to a further particular aspect, is provided a 1-deoxysphingolipid of the invention selected from:
- **a 1-deoxysphingoid base pro-drug of Formula (I):** 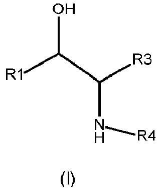 and
- **a 1-deoxyceramide of Formula (II):** 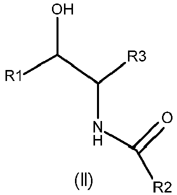 wherein R¹ is an optionally substituted C₁₁-C₁₇ aliphatic linear or isobranched chain, said C₁₁-C₁₇ aliphatic linear or isobranched chain optionally containing one or more unsaturations, including optionally substituted C₁-C₁₁ alkenyl or ω-1 alkyne groups; R² is optionally substituted C₁₃-C₂₅ fatty acid chain, containing one or more unsaturations; R³ is an optionally substituted C₁-C₆ alkyl such as methyl and R⁴ is selected from H and optionally substituted C₁-C₆ alkyl; as well as its tautomers, its geometrical isomers, its optically active forms as enantiomers, diastereomers and its racemate forms and pharmaceutically acceptable salts thereof.

According to a particular aspect, when a 1-deoxysphingoid base pro-drug of Formula (I) is administered to a subject, it is converted *in vivo* into a 1-deoxyceramide of Formula (II). According to a further particular aspect, is provided a 1-deoxysphingoid base pro-drug of Formula (I), wherein R¹ is an optionally substituted C₁₁-C₁₇ aliphatic linear, optionally containing one or more unsaturations.

According to a further particular aspect, is provided a 1-deoxysphingoid base pro-drug of Formula (I), wherein R¹ is an optionally substituted C₁₃-C₁₅ aliphatic linear or isobranched chain, optionally containing one or more unsaturations.

According to another particular aspect, a 1-deoxysphingolipid of the invention is a C14 to C20 1-deoxysphinganine, in particular a C15 to C 19 1-deoxysphinganine (e.g. C17 or C18 1-deoxysphinganine).

According to another particular aspect, a 1-deoxysphingolipid of the invention is 1-deoxyceramide of Formula (II), wherein R² is optionally substituted C₁₃-C₂₅ fatty acid chain (e.g. C₁₅), optionally containing one or more unstaurations.

According to a particular aspect, the 1-deoxysphingolipid of the invention is an *N-C12* to *N-*C36 1-deoxydihydroceramide or an *N-C12* to *N*-C36 1-deoxyceramide.

According to a particular aspect, the 1-deoxysphingolipid of the invention is an *N*-C12 to *N-*C24-deoxyceramide.

According to a particular aspect, the 1-deoxysphingolipid of the invention is an *N*-C16-deoxyceramide.

According to a further particular aspect, is provided a 1-deoxysphingoid base pro-drug of Formula (I) selected from the following group:
- **a C17 to C18 1-deoxysphinganine,** such as the C18 1-deoxysphinganine of Formula **(1):** 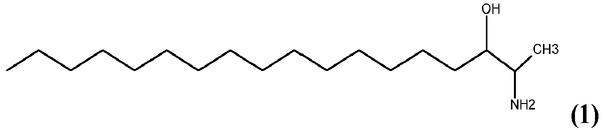 2-aminooctadecan-3-ol or the **C18 1-deoxysphinganine,** ω-1 alkyne-1-deoxysphinganine of Formula **(2):** 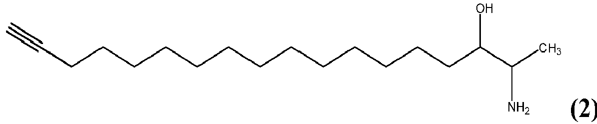 2-aminooctadec-17-yn-3-ol; or
- **a C17 to C18 1-deoxysphingosine,** in particular a C18 1-deoxysphingosine; as well as tautomers, geometrical isomers, optically active forms as enantiomers, diastereomers and racemate forms and pharmaceutically acceptable salts thereof.

According to a further particular aspect, is provided a 1-deoxysphingoid base pro-drug of Formula (I), wherein R⁴ is H.

According to a further particular aspect, is provided a 1-deoxyceramide of Formula (II), wherein R¹ is an optionally substituted C₁₁-C₁₇ aliphatic linear, optionally containing one or more unsaturations.

According to a further particular aspect, is provided a C18 1-deoxyceramide of Formula (II'): 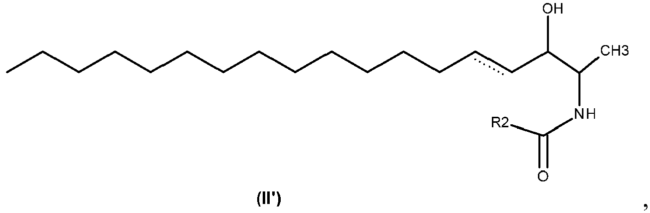 wherein R² is as defined herein and one double bond (˙˙˙) is present or not at any position on the upper aliphatic chain.

According to a further particular aspect, is provided a C18 ω-1 alkyne-1-deoxydihydroceramide of Formula (II"): 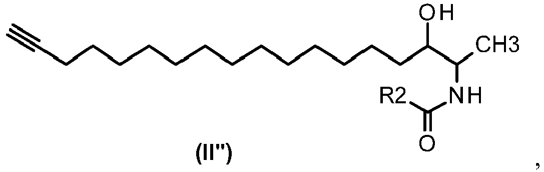 wherein R² is as defined herein.

According to a further particular aspect, is provided a 1-deoxyceramide of Formula (II) selected from the following group:
- **an *N*-C12- to *N*-C24- 1-deoxydihydroceramide,** in particular an *N*-C16 to *N*-C18 1-deoxydihydroceramide such as an ***N*-C16 1-deoxydihydroceramide** of Formula **(3):** 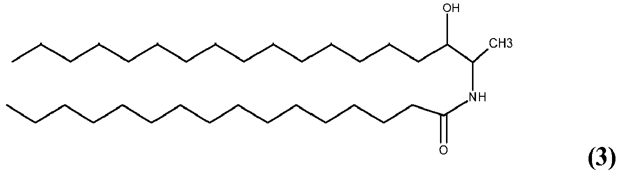 *N*-(3-hydroxyoctadecan-2-yl)palmitamide or an ***N*-C16 ω-1 alkyne-1-deoxydihydroceramide of Formula (4):** 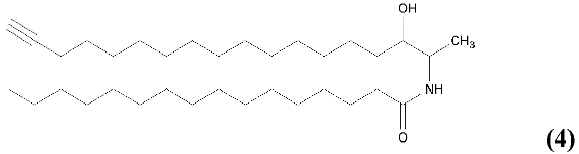 *N*-(3-hydroxyoctadec-17-yn-2-yl)palmitamide; or
- **an *N*-C12- to *N*-C24-1-deoxyceramide;** as well as tautomers, geometrical isomers, optically active forms as enantiomers, diastereomers and racemate forms and pharmaceutically acceptable salts thereof.

Compounds of the invention such as C17 iso-branched 1-deoxysphinganine are described in Hannich, 2017, Chem Sci., 8(5):3676-3686 and such as alkyne-1-deoxysphinganie in Alecu, 2017, J Lipid Res., 58(1):42-59 and encompass natural compounds (D-erythro) or non-natural compounds (L-threo), which are commercially available or can be obtained as described in Yun, 2003, J. Org. Chem., 68(20): 7675-7680 *and* Ko, 2013, J. Org. Chem., 78(2): 498-505*.* Compounds of the invention also encompass modified derivatives for conjugations with azide-reagents such as alkyne modified derivatives where the alkyne group can be attached at the end of the R¹ or R² chain for allowing click reaction such as described in *Alecu, 2017, supra.*

### Compositions

Pharmaceutical compositions of the invention can contain one or more compounds according to the invention and a pharmaceutically acceptable carrier, diluent or excipient thereof. According to a particular aspect, the anti-toxin or anti-viral effects can be achieved through the direct delivery of a 1-deoxyceramide of the invention in an appropriate delivery system (e.g. vesicles, liposomes calcium phosphate nanocomposite particles (CPNPs), linear dendritic nanoparticles (NPs), nanoemulsions, poly(ethylene oxide)-modified poly(epsilon-caprolactone) (PEO-PCL) NPs as summarized in Ma et al., 2016, Expert Opin Drug Deliv., 13(10): 1397-406).

According to another particular aspect, the anti-toxin or anti-viral effects can be achieved through the delivery of a 1-deoxysphingoid base prodrug of a 1-deoxyceramide of the invention.

According to another particular aspect, the anti-toxin or anti-viral effects can be achieved through the direct delivery of a 1-deoxyceramide of the invention.

According to a particular aspect, compositions further comprise a compound useful in a prevention and/or treatment of intoxication or viral infections by toxins or viruses. Compositions of this invention may also be formulated for parenteral administration including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water. Compositions of this invention may be formulated for inhalation, which may be in a form including, but not limited to, a solution, suspension, or emulsion that may be administered as a dry powder or in the form of an aerosol using a propellant, such as dichlorodifluoromethane or trichlorofluoromethane.

According to a particular embodiment, compositions according to the invention are for mucosal surface delivery.

In another particular aspect, compositions according to the invention are adapted for delivery by single or multiple administrations.

Alternatively, compositions of this invention may also be formulated as an aerosolable solution or an inhalable pharmaceutically acceptable composition. In such a formulation, the compound according to the invention is prepared for example as an inhalable dry powder or as an aerosolable solution. In particular, compositions suitable for nasal delivery may be formulated as drops, sprays, gels, suspensions, emulsions, microemulsions, micellar formulations, liposomal formulations, powders, microparticles and nanoparticles.

According to a particular embodiment, compositions of the invention are veterinary compositions.

Further materials as well as formulation processing techniques and the like are set out in Part 5 of Remington's "The Science and Practice of Pharmacy ", 22nd Edition, 2012, University of the Sciences in Philadelphia, Lippincott Williams & Wilkins, which is incorporated herein by reference.

According to a particular aspect, is provided a pharmaceutical composition comprising at least one 1-deoxysphingolipid according to the invention and a pharmaceutically acceptable carrier, diluent or excipient thereof, wherein said 1-deoxysphingolipid is not 1-deoxysphinganine.

The invention provides compounds of the invention, compositions thereof and methods using the same useful in the prevention and/or treatment of a medical disorder, in particular as anti-toxins and anti-viral agents for preventing and/or treating infections by toxins (from bacteria or plants) or viruses.

### Mode of administration

Compounds and compositions of this invention may be administered or delivered in any manner including, but not limited to, orally, parenterally, sublingually, transdermally, transmucosally, topically, via inhalation, via buccal or intranasal administration, or combinations thereof. Parenteral administration includes, but is not limited to, intra-venous, intra-arterial, intra-peritoneal, subcutaneous and intra-muscular.

In another particular embodiment, a compound according to the invention is administered systemically by injection.

In another particular embodiment, a compound according to the invention is administered by inhalation.

In another particular embodiment, a compound according to the invention is administered transmucosally.

In another particular embodiment, a compound according to the invention is administered intra-nasally.

The dosage administered, as single or multiple doses, to an individual will vary depending upon a variety of factors, including pharmacokinetic properties, subject conditions and characteristics (sex, age, body weight, health, and size), extent of symptoms, concurrent treatments, frequency of treatment and the effect desired.

### Combination

According to one embodiment of the invention, the compounds according to the invention and pharmaceutical formulations thereof can be administered alone or in combination with a co-agent useful in the prevention and/or treatment of a disease.

According to one aspect, compounds of the invention can be administered in combination with at least one therapeutic molecule useful in the prevention and/or treatment of bacterial intoxication or viral infections, for example a co-agent selected from known anti-toxins such as listed in Baillie, 2006, J Appl Microbiol., 101(3):594-606 or anti-viral treatments as described in Razonable 2011,Mayo Clin Proc., 86(10): 1009-1026*.*

The invention encompasses the administration of a compound of the invention wherein the compound is administered to a subject prior to, simultaneously or sequentially with a therapeutic regimen or at least one co-agent. The compound according to the invention that is administered simultaneously with said at least one co-agent can be administered in the same or different compositions and in the same or different routes of administration

### Subjects

In an embodiment, subjects according to the invention are suffering from or at risk of suffering from intoxication or viral infection by a toxin or a virus.

In a further embodiment, subjects according to the invention are suffering from or at risk of suffering from an intoxication by a toxin.

In a further embodiment, subjects according to the invention are suffering from or at risk of suffering from an intoxication by a bacterial toxin, such as anthrax toxin.

In a further embodiment, subjects according to the invention are suffering from or at risk of suffering from a viral infection.

In a further embodiment, subjects according to the invention are suffering from or at risk of suffering an infection by a Rhabdoviridae virus such as VSV or rabies virus.

### Use according to the invention

The compounds and methods according to the invention are useful in the prevention and/or treatment of intoxication or viral infection by a toxin or a virus that depend on CCT/TRiC.

In particular, the compounds and methods according to the invention are useful in the prevention and/or treatment of intoxication by bacterial toxins such as from Bacillus anthracis (Anthrax), *Vibrio cholerae* (Cholera), by *S. dysenteriae, Clostridium difficile,* and the shigatoxigenic serotypes of *Escherichia coli* (STEC) (Shiga toxin) and by plant toxins such as from *Ricinus communis* (castor beans) (ricin). Examples of viral infections according to the invention are viral infections from influenza virus, reoviruses such as Rotavirus, Hepatitis B, Hepatitis C, Epstein Barr virus, Rabies virus, Mason Pfizer monkey virus (simian retrovirus), Vesicular Stomatitis Virus and relatives (Rhabdoviridae), Flaviviridae like Dengue virus, foot and mouth, cattle, fish and other non-enveloped viruses.

References cited herein are hereby incorporated by reference in their entirety. The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description. Such modifications are intended to fall within the scope of the appended claims. The invention having been described, the following examples are presented by way of illustration, and not limitation.

### EXAMPLES

The following abbreviations refer respectively to the definitions below:
**DAPI** (4',6-diamidino-2-phenylindole); **DCM** (dichloromethane); **DMF** (dimethyl furan); **DMSO** (dimethylsulfoxyde); **EDTA** (Ethylenediaminetetraacetic acid); **EV** (empty vector); **GFP** (green fluorescent protein); **HEPES** (4-(2-hydroxyethyl)-1-piperazineethanesulfonic acid); **MOPS** ((3-(*N*-morpholino)propanesulfonic acid)); **OD₆₀₀** optical density of yeast culture measured at 600 nm wavelength; **pac** (photoactivatable and cross-linkable); **PMSF** (phenylmethylsulfonyl fluoride); **PBS** (phosphate-buffered saline); **SDS** (sodium-dodecyl sulfate); **TCEP** (tris(2-carboxyethyl)phosphine); **TES** (2-[Tris(hydroxymethyl)-methylamino]-ethanesulfonic acid); **UV** (Ultraviolet); **WT** (wildtype).

### Example 1: Effects of compounds of the invention on yeast

In order to assay the efficacy of compounds of the invention, yeast cultures were treated with a low dose of the C18 1-deoxysphinganine **(1),** for example, for a period of a few hours and then infection was measured as described below.

Cells were spotted at a ten-fold serial dilution onto rich agar media supplemented with concentrations of 1-deoxysphinganine from 5 to 20 µM. Colony size and density were recorded following 48 h incubation at 30°C. As can be seen on **Figure 1a**, yeast cells with empty vector (WT+EV) or expressing mammalian ceramide synthases 1,2 or 4 (WT+CerS1, WT+CerS2, WT+CerS4) are insensitive to 1-deoxysphinganine at the concentrations tested. Yeast expressing mammalian ceramide synthases 5 or 6 (WT+CerS5, WT+CerS6) are mildly sensitive at 10 µM 1-deoxysphinganine and highly sensitive at 20 µM 1-deoxysphinganine, whereas yeast expressing mammalian ceramide synthase 3 (WT+CerS3) are highly sensitive already at 5 µM 1-deoxysphinganine. These results show that conversion of C18 1-deoxysphinganine **(1)** into *N*-C16-C18-1-deoxydihydroceramide **(3)** leads to toxicity in yeast. **Figure 1b** represents a typical structure of yeast 1-deoxydihydroceramides (DoxDHCer) **(II")** from *Saccharomyces cerevisiae* wherein **R** is an alkyl chain with 13 to 25 carbons, which can be hydroxylated. The structure containing non-canonical **DoxSa** (in grey) corresponds to a 1-deoxyceramide of Formula (II) wherein R¹ is a C₁₅ aliphatic chain.

Therefore, this supports that the corresponding *N*-C16 1-deoxydihydroceramide **(6)** or other 1-deoxydihydroceramides could also substitute for the 1-deoxysphinganine, if applied with an adequate delivery system due to its high insolubility in water.

These experiments also show that only certain mammalian ceramide synthases are capable of converting 1-deoxysphinganine into the active compound that causes toxicity in this yeast system. The most efficient conversion was seen in the yeast strain harboring the mammalian ceramide synthase 3 (CerS3). On **Figure 1c****,** are identified by MRM-based mass spectrometry via direct infusion (see below in yeast lipid analysis) the 1-deoxydihydroceramides produced by the different strains and the quantities measured are related to the darkness of the bar. These experiments show that the acyl chain length of the 1-deoxydihydroceramide depends upon the mammalian ceramide synthase that is present but that the quantity produced cannot fully explain the toxicity results, implying that the acyl chain length also plays a role in toxicity. Therefore, efficacy of 1-deoxysphinganine will depend on the expressed ceramide synthases in the targeted tissue. When replacing 1-deoxysphinganine by specific 1-deoxydihydroceramides, the chain-length of the incorporated fatty acid will also influence the efficacy.

### Yeast strain construction

Yeast strains expressing mammalian ceramide synthases were constructed as follows: yeast codon-optimized genes of *Mus musculus* ceramide synthases 1,2,4,5,6 and *Homo sapiens* ceramide synthase 3 were synthesized by GENEART (Regensburg, Germany) containing an amino-terminal flag-tag, subcloned first into pRS424 (BamHI/EcoRI) then together with the *TDH3* promoter and *CYC1* terminator (SacI/KpnI) into integrative vectors Yiplac204(*TRP1*) or Yiplac211(*URA3*). For the wildtype strains expressing mammalian ceramide synthases strains RH2881 (WT) was transformed with linearized Yiplac204(*TRP1*) and for the different EUROSCARF single mutant strains expressing ceramide synthase 3 the EUROSCARF single mutant strains were transformed with linearized Yiplac211(*URA3*) construct.

### Yeast growth assays

Yeast strains were grown at 30°C to saturation in liquid YPD with additional uracil, adenine, and tryptophan (YPUATD). Stationary yeast cultures were diluted to OD₆₀₀=1.5 and consecutive ten-fold dilutions with double-distilled water. About 3 µl of the dilutions were pinned onto YPUATD agar plates containing 0.05% tergitol NP-40, ethanol vehicle and 0, 2.5, 5, 10, and 20 µM 1-deoxysphinganine (DoxSa). Plates were incubated for two days at 30°C or, in the case of thermo-sensitive strains, for three days at 24°C before monitoring growth.

### Yeast lipid analysis

Yeast lipid extraction and analysis was performed as previously described (da Silveira Dos Santos et al. 2014, Mol Biol Cell, 25:3234-3246; Guan et al. 2010, Methods Enzymol. 470:369-391). Yeast strains expressing the mammalian ceramide synthases were grown to OD₆₀₀=1.0 in simple YPUATD and then grown for 90 minutes in YPUATD with ethanol vehicle control or 2.5 µM 1-deoxysphinganine before metabolism was stopped with 5% TCA. Lipid extraction and targeted analysis of candidate lipid species was done as previously described (*da Silveira Dos Santos et al. 2014, supra*) based on multiple reaction monitoring (MRM-MS). Briefly, a Triversa NanoMate (Advion, Ithaca, NY, USA) was used to infuse samples onto a TSQ Vantage triple quadrupole mass spectrometer (ThermoFisher Scientific, Waltham, USA). MRM-MS was used to identify and quantify lipid species as previously described (Guan, X.L. et al. 2010). Data were converted and quantified relative to standard curves of internal standards that had been spiked in before extraction. At least two independent biological replicates were analyzed, each of which comprised up to six technical replicates. Lipid amounts were normalized by inorganic phosphate.

### Example 2: Molecular targets of compounds of the invention

In order to understand the mechanism of action of the compounds of the invention, a genetic analysis called SATAY was performed as described below in order to get indications about the molecular target, but also about the cellular pathways affected by the action of the compounds of the invention.

Cells were spotted at a ten-fold serial dilution onto rich agar media supplemented with the indicated concentrations of the C18 1-deoxysphinganine **(1).** Colony size and density were recorded following 48 h incubation at 30°C. One can see on **Figure 2****,** that all of the mutants are slightly more sensitive to 2.5 µM of the C18 1-deoxysphinganine **(1).** The results of the SATAY analysis on **Figure 2a** **and b** and the genetic interactions common to the identified hypersensitive mutant strains on **Figure 3b** suggest that 1-deoxysphingolipids could act through inhibiting proteins involved in protein folding, the actin and tubulin cytoskeletons.

### SATAY transposon screen

*Library*: SAturated Transposon Analysis in Yeast (SATAY) was performed as described before (Michel, 2017, Elife, 8:6, pii: e23570*)* with minor modifications. Briefly, BY4741 CerS3 SATAY cells (*MATa his3Δ1 leu2Δ0 met15Δ0 ura3Δ0 ade2Δ::HIS3*) were transformed with pBK257 plasmid containing the transposon. Freshly transformed cells were inoculated into 1 1 of SC-Ura (synthetic complete (0.67% yeast nitrogen base without amino acid and with amonium sulfate, 40 mg/l amino acids and bases) liquid medium without uracil containing 0.2% glucose, 2% raffinose) at OD₆₀₀ of 0.15 and grown at 30°C until saturation (OD₆₀₀ of 3-4). Then, the culture was concentrated ten times by centrifugation at 600 ×*g* for 5 min to obtain final OD₆₀₀ of 37. To induce transposition, the cells were plated onto 433 8.5-cm Petri dishes of SC-Ade agar (synthetic complete liquid medium without adenine containing 2% galactose and 2% bacto agar) and incubated at 30°C for 3 weeks. Next, colonies were scrapped using a glass rod with minimum amounts of SC liquid-Ade (synthetic complete liquid medium as above but lacking adenine and containing 2% glucose), pooled, inoculated into 1 1 of SC liquid-Ade at OD₆₀₀ of 0.125, and incubated at 30°C until OD₆₀₀ of 0.5. The library was used immediately.

*Treatment*: The cells in the library were pelleted at 800 ×*g* for 5 min, inoculated into prewarmed 500 ml of SC liquid-Ade containing solvent control or 1-deoxysphinganine at OD₆₀₀ of 0.1, and incubated at 30°C until saturation. The treatment was repeated once. Next, the cells were harvested by centrifugation at 2,500 ×*g* and 4°C for 5 min and stored at -80°C. *DNA preparation:* Genomic DNA of about 500 mg of cells was extracted by the phenol/chloroform extraction method. Next, 2 µg of genomic DNA was digested with 50 units of DpnII or NlaIII at 37°C for 24 h. The enzymes were then heat inactivated at 65°C for 20 min. The DNA fragments were circularized with 25 Weiss units of T4 Ligase at 22°C for 6 h. The circularized DNA molecules were precipitated with 0.3 M of sodium acetate pH 5.2, 1 ml of ethanol, and 5 µg linear acrylamide (Ambion #AM9520) at -20°C overnight. Then, DNA was pelleted at 16,100 ×*g* and 4°C for 20 min, washed with 1 ml of 70% ethanol, and dried at 37°C for 10 min. Next, transposon fragments were amplified with Taq polymerase (New England Biolabs). The PCR products were then purified with the PCR clean-up/gel extraction kit (Macherey-Nagel) according to the manufacturer instruction, with the following modifications. DNA was bound to the column by centrifugation at 3,000 ×*g* for 30 s. Then, 30 µl of elution buffer (10 mM Tris-HCl pH 8.5, 0.1% (^{v}/ᵥ) Tween® 20) was applied to the column, incubated for 3 min, and eluted by centrifugation at 11,000 ×*g* 20°C for 1 min. The eluate was reapplied to the column and a second elution was performed under the same conditions.

*Deep sequencing*: Equal amounts of DNA from DpnII- and NlaIII-digested samples were pooled and sequenced using MiSeq v3 chemistry, according to the manufacturer instruction.

### Target identification

*Probe:* A bio-orthogonal 1-deoxydihydroceramide analogue was synthesized from 1-deoxysphinganine and C15 pac-fatty acid from Haberkant, 2013, Angew Chem Int Ed Engl., 52(14):4033-8 via amide formation. To this end, 1-deoxysphinganine was coupled in dichloromethane with pac acyl chloride, which was formed by treating the pac fatty acid with oxalyl chloride in DCM in the presence of catalytic amounts of DMF. The resulting pac-1-deoxydihydroceramide (*N*-((*2S*,*3R*)-3-hydroxyoctadecan-2-yl)-9-(3-(pent-4-yn-1-yl)-3*H-*diazirin-3-yl)nonanamide) **(****Figure 4****)** was used for identification of protein targets by chemoproteomics. Multilamellar vesicles of pac-1-deoxydihydroceramide were generated by mixing egg yolk sphingomyelin (Sigma #S0756) and pac-1-deoxdihydroceramide in a ratio of 95 to 5 in a glass tube, drying under nitrogen flow and resuspending in MiliQ water by vortexing, incubation at 60°C for 10 minutes and sonication in a sonication bath for 30-60 minutes.

*Proteome:* Lysates were prepared from 80 OD₆₀₀ of early log-phase yeast culture. Cells were harvested by 5-minute centrifugation at 1200 ×*g* and 4°C, washed and resuspended in lysis buffer (50 mM HEPES-NaOH, pH 7.5, 100 mM NaCl, 1 mM of PMSF, Complete protease cocktail inhibitor without EDTA), and broken with chilled glass beads using a genie disruptor for 10 minutes at 4°C. Crude lysate was cleared by 300 ×*g* centrifugation for 5 minutes and nuclei were removed at 13000 ×*g* for 10 min at 4°C. Membranes were removed at 100000 ×*g* for 60 minutes at 4°C and final cytosolic supernatant was used for cross-linking. *Chemoproteomics*: 1.5 mg cytosolic fraction was diluted in 645 µL PBS containing 0,5% of digitonin and 6.45 µL multilammellar vesicles were added followed by vortexing and ambient incubation in the dark. Protein cross-linking was performed on ice for 5 minute by UV (365 nm) irradiation. Reaction of the alkyne group with biotin-azide was performed with 2x excess of biotin reagent in 1 mM TCEP and 100 µM Tris(benzyltriazolylmethyl)amine and 1 mM CuSO₄ for 60 min at room temperature in the dark. To remove the reagent 4 volumes methanol and 1 volume chloroform were added. Following centrifugation at 14000 ×*g* for 5 minutes at 4°C, top and bottom liquid phases were removed and protein interphase was dried. Proteins were resuspended in PBS containing 2% SDS, sonicated and cleared from insoluble material by a 5-minute centrifugation at 4700 g. Proteins were diluted to 0.2% SDS with PBS, bound to streptavidin beads (Pierce, ThermoFisher #88816) and incubated overnight in the dark at room temperature. Beads were washed with PBS containing 1% SDS, followed by PBS and water. Beads were further treated with 6 M urea in PBS containing 10 mM neutralized TCEP and rotated for 30 minutes in the dark at room temperature, followed by treatment with 25 mM iodoacetamide during another 30-minute rotation in the dark at room temperature. Beads were washed with PBS and resuspended in 2 M urea buffered with NH₄HCO₃ (pH 8). Proteins were digested on the beads by incubation with Trypsin in the presence of 1 mM CaCl₂ at 37°C on a thermoshaker in the dark overnight. Samples were desalted and analyzed on a Orbitrap Fusion Tribid mass spectrometer. Data analysis was done using the MaxQuant software.

The SATAY analysis in ceramide synthase 3 expressing yeast (**Figure 2****)**, helps reveal yeast mutants that show resistance or hypersensitivity to 1-deoxysphingolipids of the invention, mainly *N*-C26 1-deoxydihydroceramide and possibly also 1-deoxysphinganine. In this case, the results from the resistant mutants further confirmed that the C18 1-deoxysphinganine **(1)** must be converted to the corresponding *N*-C26-C18-1-deoxydihydroceramide for toxicity and the hypersensitive mutants provided insights into the pathways affected by compounds of the invention, in particular to define the pathways that are negatively impacted by the compounds of the invention. One group of hypersensitive genes (DYN1, BIK1, NUM1, PAC1, PAC11, KIP2) are involved in the pathway of nuclear migration **(****Figure 2****).** Even though the increase in sensitivity is weak **(****Figure 2b****),** there are consistent results with a large number of mutants in the pathway. These results suggest that 1-deoxysphingolipids cause a defect in nuclear migration. This would be consistent with an inhibition of a microtubule related process. Further negative genetic interaction analysis of the hypersensitive mutants **(****Figure 3a****)** identifies mutants involved in actin cables, microtubules and protein folding **(****Figure 3b****).** This suggests a general problem of protein folding, most likely affecting actin and tubulin.

While genetic analysis helped us identify the pathway targeted by 1-deoxysphingolipids of the invention a photo-cross-linkable bio-orthogonal 1-deoxydihydroceramide analogue was used to identify the direct molecular targets. The photo-activatable and click-able (pac-) 1-deoxydihydroceramide of Formula (II) wherein R¹ is a C₁₅ linear aliphatic chain, R³ is a methyl was synthesized by coupling the 1-deoxysphinganine in dichloromethane with pac acyl chloride, which was formed by treating the pac fatty acid with oxalyl chloride in DCM in the presence of catalytic amounts of DMF, as represented by a compound of Formula (II"') on **Figure 4** where, in the R² chain, were introduced **(i)** a diazirine group to allow photo-activated cross-linking to protein targets and **(ii)** an alkyne group to allow functionalization by biotinylation via click chemistry for use as a bio-orthogonal probe for chemoproteomic analysis. Shortly, yeast lysates were prepared and a cytosolic fraction was isolated by differential centrifugation. The photo-cross-linkable 1-deoxydihydroceramide of Formula (II"') was incubated with the cytosolic fraction and cross-linked to a number of interacting protein targets including all subunits of the essential protein chaperonin complex called CCT/TRiC. Pull-down of cross-linked protein targets via biotin-streptavidin purification identified all 8 subunits of CCT/TRiC, all of which are encoded by essential genes in yeast. Consistent with the genetic analysis, this complex, indeed, is involved in folding of actin and microtubule monomers which make up the cytoskeletal structures in the cell. The fact that all the subunits are essential can explain why there are no resistant mutants outside the ceramide biosynthetic pathway in the SATAY analysis.

To provide additional evidence that CCT/TRiC is a target that is negatively impacted by 1-deoxysphingolipids, yeast strains harboring mammalian CerS3 ensuring synthesis of the *N-*C26 1-deoxydihydroceramide **(6)** and conditional yeast mutants in CCT/TRiC (*tcp1-1* and *cct4-1*) were created. Wilt-type (WT) (BY4741) and thermo-sensitive yeast mutants expressing control constructs or mammalian ceramide synthase 3 (CerS3) were grown at different densities on rich medium with 0.05% tergitol and solvent control (EtOH) or increasing concentrations of the C18 1-deoxysphinganine **(1)** and incubated for 3 days at 24°C. Temperature sensitive CCT/TRiC mutants expressing CerS3 showed hypersensitivity to the C18 1-deoxysphinganine **(1)** compared to WT expressing CerS3 or control thermo-sensitive strains (**Figure 5**). In particular, thermo-sensitive mutants of CCT/TRiC subunits, *tcp1-1* and *cct4-1,* are hypersensitive to the *N-C26-C*18-1-deoxydihydroceramide which is consistent with the hypothesis that 1-deoxydihydroceramides inhibit CCT/TRiC. Hypersensitivity arises because these mutants already have a compromised CCT/TRiC and the corresponding *N*-C26-C18-1-deoxydihydroceramide produced by CerS3 further compromises the already reduced activity of the mutant CCT/TRiC. From this finding, it can be concluded that the relevant cellular target of 1-deoxysphingolipids of the invention is the CCT/TRiC.

### Example 3: Effects of compounds of the invention on bacterial toxins and viruses

C18 1-deoxysphinganine **(1)** was assayed for its toxicity to the tissue culture cell lines that will be to test for anthrax toxin sensitivity and viral infection, as follows.

For anthrax toxin activity, RPE1 cells were used while HeLa MZ cells were used for viral infection. Cell lines were incubated with different concentrations of **(1)** overnight and cellular toxicity of the compound of the invention was determined. Then, concentrations up to 1 µM that were not toxic to RPE1 cells were used for anthrax toxin activity assays while for the HeLaMZ cells, a range of 0.2 to 2 µM were tested for viral infections.

### Activity towards anthrax toxin

Anthrax toxin has three functional units, protective antigen (PA) that binds to the cell surface receptor, lethal factor (LF), which cleaves and downregulates MEK1 and edema factor that upregulates cAMP. After binding to the receptor, PA is cleaved, then forms heptamers and the toxin is endocytosed. Later, lethal and edema factors are delivered to the cytoplasm where they can perform their toxic functions. To test whether a compound of the invention would be capable of blocking anthrax toxicity, RPE1 cells were incubated with the PA and LF anthrax toxin in presence of 1 µM (sub-lethal concentration) of the C18 1-deoxysphinganine **(1)** for increasing times, as described below:
Immortalized human epithelial cell line RPE1 cells (ATCC #CRL-4000) was cultured in Dulbecco's Modified Eagle Medium (DMEM) GlutaMAX supplement (Life Technologies #31966) + 10% fetal bovine serum (FBS) (Pan Biotech #P30-3303M) + 1% Penicillin-Streptomycin (10 000U/Ml) (Gibco #15140122). Anthrax toxin was prepared as described by Leppla, 1988, Methods Enzymol., 165:103-16 including wildtype PA and LF. Antibody against anthrax PA (Liu, 2001, J Biol Chem., 276(21):17976-84) was prepared as described. An antibody against the N-terminal peptide of MEK1 was produced as described in Abrami, 2010, PLoS Pathog. 6(3):e1000792; anti-actin from Millipore (MAB1501-RRID:AB-_2223041); anti-CCT5 from Abcam (Ab-129016- RRID:AB_11154964), HRP secondary antibodies were from Pierce.

*Anthrax toxin assay:* RPE1 cells were treated overnight with vehicle control (DMSO) or 1 µM of the C18 1-deoxysphinganine **(1)** in complete medium followed by incubation with a mix of 500 ng/ml PA83 and 50 ng/ml LF for 1 h at 4°C and different times at 37°C and cell extracts (40 µg of proteins) were analyzed by SDS-PAGE. PA processing and LF activity is monitored by western blot using an antibody against the N-terminus of MEK1 (MEK1(N)), using actin as equal loading control for normalization. PA monomer, SDS-resistant heptamer and N-terminus of MEK1 (MEK1(N)) are revealed.

*Cell extract and SDS-PAGE analysis:* Cells were harvested, washed with PBS and homogenized by passage through a 22 gauge injection needle in HB (HB: 2.9 mM imidazole and 250 mM sucrose, pH 7.4) containing the Roche mini tablet protease inhibitors cocktail following manufacturer's instructions. Protein quantification was done with Pierce BCA kit. Proteins were loaded at 40 µg protein/lane and separated on a 4-20% acrylamide precast Novex gel : 4-20% acrylamide gel, Novex Tris-Glycine Mini gels Wedge well, 10-well (Life Technologies #XP04200) using Laemmli sample buffer (buffer 2x: 120 mM Tris-HCl, pH 6.8, 20% glycerol, 4% SDS, 0.02% bromophenol blue., 10% beta-mercaptoethanol) and SDS-PAGE running buffer (1.44% Glycine, 0.1% SDS in 50 mM Tris).

As it can be seen on Western Blot analysis from **Figure 6****,** in both control and treated samples, PA is processed and cleaved from PA83 to PA63 and forms heptamers and this process is unaffected by the presence of the C18 1-deoxysphinganine. On the other hand, in absence of the C18 1-deoxysphinganine, MEK1(N) is cleaved, disappearing from the detection system within 90 to 120 min, whereas, in the presence of the C18 1-deoxysphinganine, LF activity is prevented and MEK1(N) is stabilized showing that the compound of the invention blocks one of the essential steps in anthrax toxicity. No other compound seems to have been described to be effective at this late step in anthrax toxicity as most anthrax anti-toxin therapies are antibody based and target very early steps of toxin binding to the receptor and internalization (Baillie, 2006, J Appl Microbiol., 101(3):594-606).

### Activity towards Vesicular Stomatitis Virus (VSV)

VSV affects cattle and other livestock and belongs to the same family of viruses as rabies. HeLaMZ cells were incubated with increasing concentrations of C18 1-deoxysphinganine (1) overnight as described below:
HeLaMZ (10⁴ cells/well) were seeded in 96-well Imaging Microplates (BD Falcon™# 353219) the day before the assay. After 6h, C18 1-deoxysphinganine was added from a stock solution (10 mM in EtOH abs) to reach a final concentration of 0, 0.2, 0.5, 1, and 2 µM and cells were incubated with the compound overnight.

A recombinant vesicular stomatitis virus expressing GFP-tagged P-protein (VSV-PeGFP) was used to infect cells (Das et al., 2006, Journal of virology 80:6368-6377*;* Luyet et al., 2008, Traffic, 9:2279-2290): virus binding was performed for 45 min at 4°C in G-MEM (Sigma G6148) containing 10 mM TES, 10 mM MOPS, 15 mM Hepes, 2 mM NaH₂PO₄ hydrated, 35 mg/L NaHCO₃, pH 7.4. Cells were then incubated for 3.5h at 37°C (Le Blanc et al., 2005, Nat Cell Biol, 7:653-664*;* Bissig at al., 2013, Dev Cell, 25:364-373) with the VSV viral particles encoding for GFP-fusion of the P-protein. The virus was used at a low physiologically relevant Multiplicity of infection (MOI), so that approximately 50% cells only were infected in controls. After 3 hours of incubation, infection was quantified as GFP-positive cells. Cells were fixed with 3% paraformaldehyde for 20 min at room temperature and stained with Hoechst (1:5000 in PBS with 0.05% saponin). Image acquisition was performed immediately after staining using the IXM™ microscope (Molecular Devices), with the 20X objective. Nine images were captured for each well and were analyzed using the MetaXpress Custom Module editor software. In the analysis, cell images were segmented using the DAPI (Hoechst) channel. Cells with a GPF signal set at an arbitrary threshold over background were considered as infected. Data are expressed as a percentage of infected cells normalized to the infection of untreated which was set to 100%.

Cell count measurements by DAPI-based nuclei count shown in **Figure 7 (b)****,** indicate that the compound of the invention was not toxic at these concentrations. However, starting at 500 nM, the compound reduced viral infection and at 1 µM, the reduction of viral infectivity was substantial **Figure 7 (a)****.** These experiments confirm that C18 1-deoxysphinganine inhibits viral infection and in combination with the effects on anthrax toxin, further support the idea that those compounds act via inhibition of the CCT/TRiC.

Analogues of C18 1-deoxysphinganine **(1)** were tested in the same conditions and C18 ω-1 alkyne-1-deoxysphinganine **(2)** showed similar inhibition of viral infections as C18 1-deoxysphinganine **(1)** (Figure 8 a). **(1)** starts to show some toxicity at 1µM, while (**2)** shows very little toxicity as can be seen in a more stable cell number (Figure 8 b).

Those findings further support that compounds of the invention should be useful against intoxication or viral infections that use the endocytic pathway of the host, in particular through the action of the CCT/TRiC pathway.

## Claims

1. A 1-deoxysphingolipid, as well as pharmaceutically acceptable salts thereof, for use in the prevention and/or treatment of toxin-mediated infection or intoxication involving bacterial or plant toxins or viral infections.

2. A 1-deoxysphingolipid for use according to claim 1, wherein the 1-deoxysphingolipid is a 1-deoxyceramide or a 1-deoxysphingoid base prodrug thereof.

3. A 1-deoxysphingolipid for use according to claim 1 or 2, selected from
- a 1-deoxysphingoid base pro-drug of Formula (I): 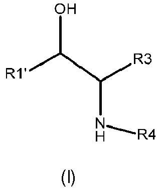 and
- a 1-deoxyceramide of Formula (II): 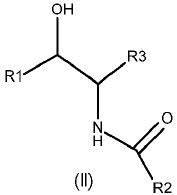 wherein R¹ is an optionally substituted C₁₁-C₁₇ aliphatic linear or isobranched chain, said C₁₁-C₁₇ aliphatic linear or isobranched chain optionally containing one or more unsaturations, including optionally substituted C₁-C₁₁ alkenyl or ω-1 alkyne groups; R² is optionally substituted C₁₃-C₂₅ fatty acid chain, containing one or more unsaturations; R³ is an optionally substituted C₁-C₆ alkyl such as methyl and R⁴ is selected from H and optionally substituted C₁-C₆ alkyl; as well as its tautomers, its geometrical isomers, its optically active forms as enantiomers, diastereomers and its racemate forms and pharmaceutically acceptable salts thereof.

4. A 1-deoxysphingoid base pro-drug of Formula (I) for use according to claim 3, wherein R⁴ is H.

5. A 1-deoxysphingoid base pro-drug of Formula (I) for use according to claim 3 or 4, wherein R¹ is an optionally substituted C₁₁-C₁₇ aliphatic linear chain, optionally containing one or more unsaturations.

6. A 1-deoxysphingoid base pro-drug of Formula (I) for use according to any one of claims 3 to 4, wherein said 1-deoxysphingoid base pro-drug is a C14 to C20 1-deoxysphinganine, in particular a C15 to C 19 1-deoxysphinganine(e.g. C17 or C18 1-deoxysphinganine).

7. A 1-deoxyceramide of Formula (II) for use according to any one of claims 3, selected from a 1-deoxydihydroceramide and a 1-deoxyceramide; as well as tautomers, geometrical isomers, optically active forms as enantiomers, diastereomers and racemate forms and pharmaceutically acceptable salts thereof.

8. A 1-deoxyceramide for use according to any one of claims 7, selected from an *N*-C12 to *N*-C36 1-deoxydihydroceramide and an *N-C12* to *N-C36* 1-deoxyceramide, in particular an *N*-C12 to *N*-C24 1-deoxydihydroceramide.

9. A 1-deoxyceramide for use according to any one of claims 2 to 3, or 7 to 8, wherein said 1-deoxyceramide is of Formula (II'): 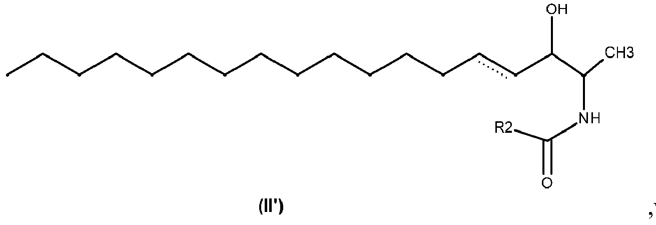 wherein one double bond (˙˙˙) is present or not at any position on the upper aliphatic chain, or of Formula (II"): 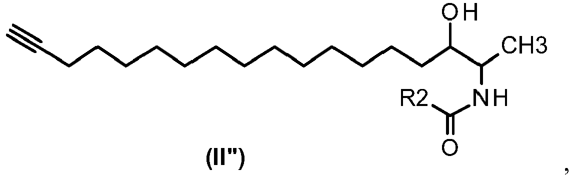 wherein R² is as defined in any one of the preceding claims.

10. A 1-deoxysphingolipid for use according to any one claims 1 to 3, selected from:
- **a C17 to C18 1-deoxysphinganine,** such as the C18 1-deoxysphinganine of Formula **(1):** 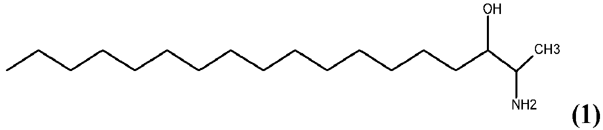 2-aminooctadecan-3-ol or the **C18 1-deoxysphinganine,** ω-1 alkyne-1-deoxysphinganine of Formula **(2):** 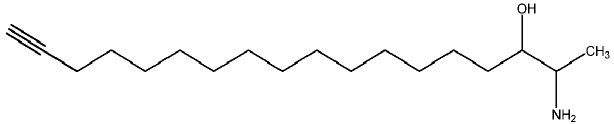 2-aminooctadec-17-yn-3-ol; or
- **a C17 to C18 1-deoxysphingosine,** in particular a C18 1-deoxysphingosine; or
- **an *N*-C12- to *N*-C24- 1-deoxydihydroceramide,** in particular an *N*-C16 to *N*-C18 1-deoxydihydroceramide such as an ***N*-C16 1-deoxydihydroceramide** of Formula **(3):** 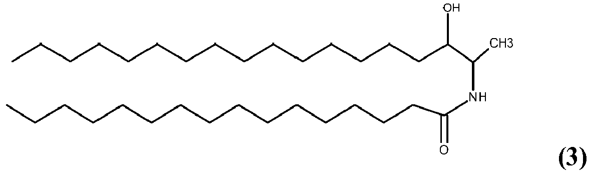 N-(3 -hydroxyoctadecan-2-yl)palmitamide; or an *N-C16* **ω-1 alkyne-1-deoxydihydroceramide** of Formula **(4):** 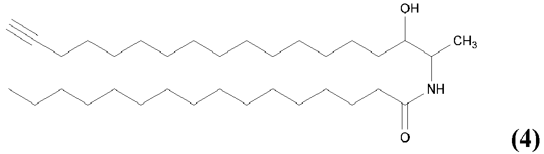 *N*-(3-hydroxyoctadec-17-yn-2-yl)palmitamide; or
- **an *N*-C12- to *N-C24-* 1-deoxyceramide;** as well as tautomers, geometrical isomers, optically active forms as enantiomers, diastereomers and racemate forms and pharmaceutically acceptable salts thereof.

11. A pharmaceutical composition comprising at least one compound described in anyone of claims 1 to 10, in combination with at least one agent useful in the preventing and/or treating toxin-mediated infections orintoxication involving bacterial or plant toxins, or infection by viruses and a pharmaceutically acceptable carrier, diluent or excipient thereof.

12. A 1-deoxysphingolipid for use according to any one of claims 1 to 10, wherein the intoxication is an intoxication by a bacterial toxins such as from *Bacillus anthracis* (Anthrax), *Vibrio cholerae* (Cholera), *S. dysenteriae, Clostridium difficile*, the shigatoxigenic serotypes of *Escherichia coli* (STEC) (Shiga toxin) and by plant toxins such as from *Ricinus communis* (castor beans) (ricin).

13. A 1-deoxysphingolipid for use according to any one of claims 1 to 10, wherein viral infection is a viral infection from reoviruses such as Rotavirus, Hepatitis B, Hepatitis C, Epstein Barr virus, Rabies virus, Mason Pfizer monkey virus (simian retrovirus), Vesicular Stomatitis Virus and relatives (Rhabdoviridae), foot and mouth, cattle, fish and other nA 1-deoxysphingolipid for use according to any one of claims 1 to 10, wherein viral infection is a viral infection from reoviruses such as Rotavirus, Hepatitis B, Hepatitis C, Epstein Barr virus, Rabies virus, Mason Pfizer monkey virus (simian retrovirus), Vesicular Stomatitis Virus and relatives (Rhabdoviridae), foot and mouth, cattle, fish and other non-enveloped viruses such as Noro virus and papillomaviruseson-enveloped viruses such as Noro virus and papillomaviruses.

14. A method of sterilisation and/or decontamination or preventing contamination with toxins or viruses, medical material intended for medical, aesthetic or hygiene practice or preparations intended for human or veterinary use comprising the step of contacting said material, room or preparations with a 1-deoxysphingolipid as described in any one of claims 1 to 10, or a composition thereof

15. An *ex vivo* method of inhibiting virulence of bacteria and/or viruses, said method comprising the step of contacting a material or a solution at risk being contaminated by said bacteria and/or viruses with a 1-deoxysphingolipid as described in any one of claims 1 to 10, or a composition thereof
